# EUROPEAN PATENT SPECIFICATION

(11) **EP 1 741 700 B1**
(45) Date of publication and mention of the grant of the patent: **24.12.2008**
(21) Application number: 06116752.4
(22) Date of filing: 06.07.2006
(51) Int. Cl.: C07D 209/88

(54) **Process for the preparation of carvedilol**
Verfahren zur Herstellung von Carvedilol
Procédé de préparation de carvedilol

(30) Priority: 06.07.2005 IN MU08072005
(43) Date of publication of application: 10.01.2007
(73) Proprietor: IPCA Laboratories Limited, Charkop, Kandivali, 400 067 Mumbai (IN)
(72) Inventor: Kumar, Ashok, 400 067, Mumbai (IN); Saxena, Ashvini, 457 002, Madhya Pradesh (IN); Bhattacharyya, Anindya, 457 002, Madhya Pradesh (IN); Singh Sengar, Amit Vikram, 457 002, Madhya Pradesh (IN); Pathak, Gunjan Pramod, 457 002, Madhya Pradesh (IN); Soudagar, Satish Rajanikant, 400 067, Mumbai (IN); Mathur, Pramil Kumar, 400 067, Mumbai (IN); Nijasure, Avinash Manohar, 400 067, Mumbai (IN); Salunke, Sanjukumar Motiram, 400 067, Mumbai (IN); Gautam, Prashant, 400 067, Mumbai (IN); Ramsingh, Thakur Gajendrasingh, 400 067, Mumbai (IN); Jadhav, Dilip Uttam, 400 067, Mumbai (IN)
(74) Representative: Brand, Thomas Louis

(56) References cited:
- EP-A- 0 004 920
- WO-A-20/04094378
- WO-A-20/04113296

## Description

The present invention relates to a preparation method for 1-(9H-Carbazol ― 4 ― yloxy) - 3 - [[2 ― (2-methoxy phenoxy) ethyl] amino]-2-propanol and its salts in higher purity.

The pharmaceutically valuable compound 1-(9H-Carbazol - 4 - yloxy) - 3 - [[2 - (2-methoxy phenoxy) ethyl] amino]-2-propanol, also known as Carvedilol under the international proprietary name, is a non - selective β - adrenergic blocking agent with vasodilating activity. Carvedilol is represented with the following structural formula:

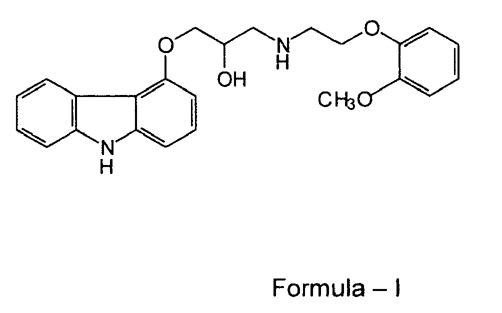

The compound carvedilol has one chiral carbon atom and hence it can exist in either as its stereoisomers or in its racemic form.

Carvedilol was reported for the first time in EP 000 4920, wherein the compound 4- (2,3- epoxy propoxy) carbazole of Formula - II is reacted with 2 - (2- methoxy-phenoxy) ethylamine of Formula - III to prepare the carvedilol as per the reaction sequence shown in scheme- I:

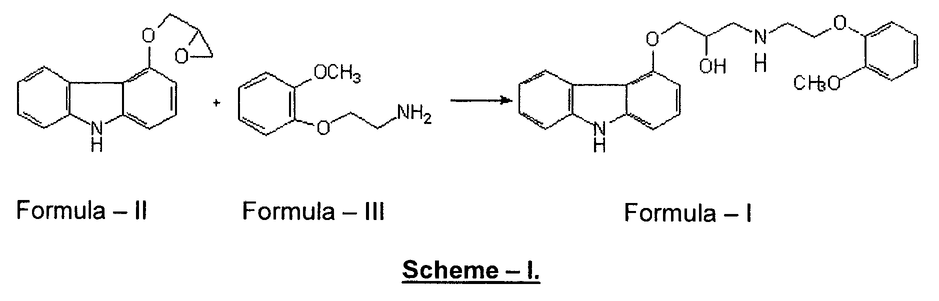

The known processes produce carvedilol in lower yield with formation of a bis impurity of Formula - IV, which in most cases cannot be avoided, in about 10-15% of the total product. This necessitates additional purification procedures for the isolation of carvedilol free from contamination of high percentage of bis-impurity and therefore the process may not be ideal for industrialization.

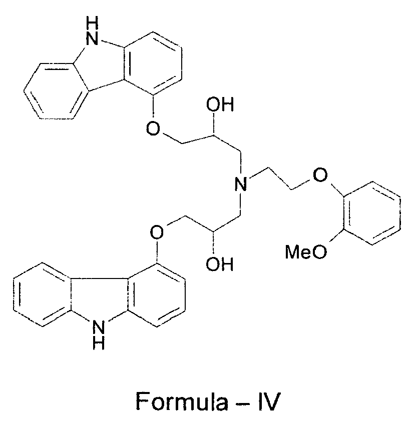

EP 0918 055 discloses a process by which the formation of bis impurity is avoided by reacting a benzyl protected 2- (2- methoxy phenoxy) ethylamine (Formula V) with 4- (2,3- epoxy propoxy) carbazole. The intermediate benzyl carvedilol (Formula VI) has to be debenzylated before isolating the product carvedilol. This process introduces two new steps namely, benzyl protection of 2-(2- methoxy phenoxy) ethylamine and deprotection of the benzyl carvedilol obtained after the condensation reaction. In addition, the process involves debenzylation of the protected moiety by employing either costly metal catalyst or hydrazine hydrate in the synthesis of carvedilol affecting the economy of the process.

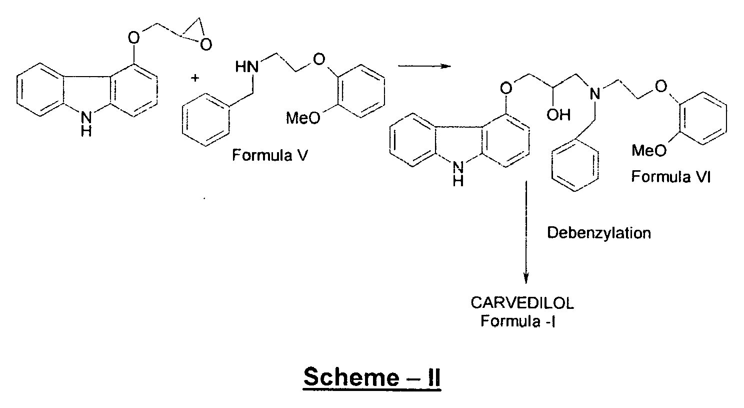

Yet another report, WO 01/87837 describes the preparation of carvedilol from 4-hydroxy carbazol by reacting it with 5 -chloromethyl - 3 - [2 - (2 - methoxy phenoxy) ethyl] - oxazolidin - 2 - one (Formula - VII) to yield oxazolidine derivative of Formula - VIII as expressed in scheme III. This intermediate is further hydrolyzed to get carvedilol. The preparative process for the intermediate of Formula -VII involves various stages, such as reacting 1,3 - dichloropropan - 2 - ol with phenyl chloroformate, which is made to react with 2-[2― (methoxy phenoxy)] ethyl amine hydrochloride to yield [2 - (2- methoxy phenoxy) ― ethyl] carbamic acid ―2-chloro ―1 ― chloromethyl - ethyl ester. The ester formed is cyclised to get compounds of Formula - VII. In order to produce carvedilol, free of bis impurity, the process introduces number of additional steps making it cumbersome for the industrial production of the compound.

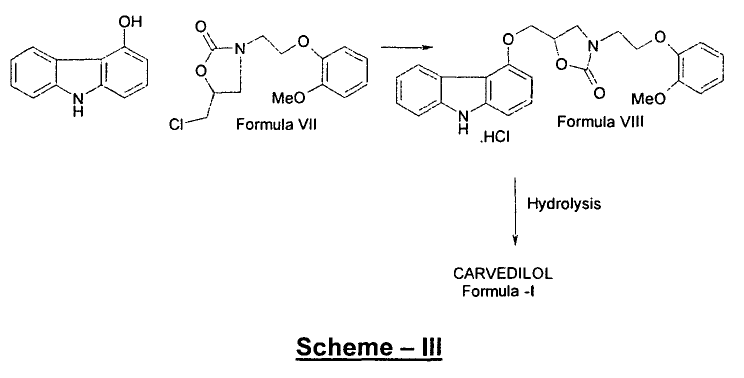

Yet another improvement reported in WO 02/00216 describes the preparation of carvedilol from 4- (2, 3- epoxy propoxy) carbazole (Formula ― II) with 2 - (2-methoxy phenoxy) ethylamine (Formula - III). The process uses a large molar excess of intermediate of Formula III in the range of 2.80 moles to 100 moles per 1.0 mole of 4- (2,3- epoxy propoxy) carbazole (Formula - II) to avoid the formation of bis impurity (Formula - IV). The application further discloses the process for isolation of carvedilol as crystalline Hydrochloride, Hydrochloride Hydrate, and methyl ethyl ketone solvate. The use of large excess of 2 - (2-methoxy phenoxy) ethylamine (Formula III) makes the process uneconomical and requires the excess reactant to be recovered and recycled to make the process economically successful.

Further, WO 2004/094378 describes a process for preparation of carvedilol Form - II by reacting 4- (2,3- epoxy propoxy) carbazole (Formula - II) with 2 - (2-methoxy phenoxy) ethylamine (Formula - III) in solvents like monochlorobenzene, monoglyme or mixtures thereof. The application does not mention about the bis impurity formed during the condensation stage.

It has been a long-standing need in the industry to provide an economical process for preparation of carvedilol with control over the formation of bis impurity of Formula IV.

After thorough experimental studies the present inventors have come up with a process where the problem of formation of bis impurity is limited to acceptably low amount without using large excess of reactants or adding new reactants / reagents or introducing extra process steps in the condensation reaction of compounds of Formula II & III, which is the subject of the present invention.

The main objective of the present invention is to provide an economical and industrially feasible process for the preparation of carvedilol.
Yet another objective of the present invention is to minimize the formation of bis impurity and isolate carvedilol free from contaminating bis-impurity of formula IV in high purity and yields.

Accordingly a process is provided for the preparation of carvedilol which comprises reacting 4- (2,3- epoxy propoxy) carbazole (Formula ― II) with 2 - (2-methoxy phenoxy) ethylamine (Formula - III) in dimethyl sulfoxide at a temperature ranging from about 50 to 100 °C to form a reaction mass containing carvedilol with minimum bis-impurity. The reaction in these selected solvents minimizes the formation of the bis-impurity to less than about 7%. The invention also provides an isolation procedure for obtaining carvedilol substantially free of its bis-impurity of Formula IV.

Thus, the present invention provides a process for purification of carvedilol containing the known bis impurity (Formula IV) by subjecting the crude carvedilol to either repeatedly leaching in toluene or suspending/dissolving the contaminated carvedilol into a water immiscible organic solvent, washing the organic phase with an aqueous acid till the pH of the washings is in the range of 7.0 to 8.0; further, adding an acid to organic layer to attain a pH of 4 to 5, thereby selectively precipitating the carvedilol as an acid salt, isolating carvedilol after an acid-base treatment, which is further leached in toluene, water or mixture thereof; and finally crystallizing the carvedilol from ethyl acetate to obtain carvedilol substantially free of bis-impurity.

The process of the present invention is described herein after in more details substantiating various embodiments and conditions of reaction for better understanding of the invention.

The process of the present invention describes the preparation of carvedilol of Formula I by reacting 4 - (2,3- epoxy propoxy) carbazole (Formula - II) with 2 - (2- methoxy phenoxy) ethylamine (Formula - III) in presence of DMSO. The reaction carried out in DMSO has found to limit the formation of the bis-impurity to about 5 -7 % in comparison with 10-20% in reported solvents like alcohols, acetonitrile, ethoxyethanol, hydrocarbon solvents etc. or in neat reaction.

The reaction is preferably performed by heating the reactants in the said solvent medium and the preferred temperature for carrying out the reaction is by maintaining the reaction mass at 68 - 72°C. The reaction normally completes in a span of 15 - 20 hours. 2 - (2- methoxy phenoxy) ethylamine reactant used in the reaction is preferably in slight excess in the range of 1.5 to 2.5 molar equivalents relative to the compound 4 - (2,3- epoxy propoxy) carbazole.

In this process, the work up and isolation of the product formed is carried out either by quenching the mass directly in water or the solvent is first distilled and then it is quenched in water and extracted the free base in organic solvent like dichloro methane. The preferred method of work up is to quench the reaction mixture in water without distilling the solvent and extracting the solution with solvent dichloro methane.

Further, the carvedilol reaction mixture is purified from excess reactant and bis-impurity by the following procedure. The organic solvent extract obtained above is washed one or more times till the pH of the last washings is reached to 7.0 - 7.5 with the help of an aqueous acid solution, and the aqueous layer is separated off. The adjustment of pH to 7.0 - 7.5 and separation of aqueous layer is necessary to remove the contamination of 2 - (2- methoxy phenoxy) ethylamine (Formula - III) and other impurities. The aqueous acid used for the pH adjustment is selected from hydrochloric acid, sulphuric acid, hydrobromic acid, phosphoric acid, *p*-toluene sulphonic acid and acetic acid. After removing the aqueous layer, the pH of the organic layer is adjusted to 4 to 4.5 using an acid, preferably with the same acid to selectively precipitate carvedilol as an acid salt. The preferred acid for the pH adjustment is dilute hydrochloric acid, sulphuric acid, *p*-toluene sulphonic acid and acetic acid. The acids used to form salt of carvedilol include the above described acids as well as sulphonic acid or dibasic organic acids. The sulphonic acid is selected form methane sulphonic acid, p-toluene sulphonic acid and benzene sulphonic acid. The dibasic acids include succinic acid, phthalic acid, maleic acid, malonic acid etc. Most of the bis-impurity is removed by forming the salt of carvedilol in this isolation step. The precipitated carvedilol salt is then subjected to an acid-base treatment in an organic solvent for the isolation of crude carvedilol free base. The base used for liberating carvedilol from the corresponding acid salt is selected from aqueous ammonia solution, sodium bi carbonate, sodium carbonate, and triethyl amine.

The crude carvedilol isolated is leached in solvent selected from toluene, water or its mixture thereof and stirred at 25 - 60°C and filtered. The preferred solvent for leaching is a mixture of toluene and water. If necessary this leaching procedure is repeated to get carvedilol substantially free of bis-impurity.

The crude carvedilol is crystallized by dissolving in ethyl acetate at an elevated temperature of 76 - 80°C to get a clear solution. The carvedilol solution is optionally treated with an adsorbent and filtered to remove the adsorbent, concentrate the solution to 1/3^{rd} of the volume and subsequently cooled to 20 - 30°C to crystallize out the pure product. The preferable concentration of carvedilol relative to the solvent is in an amount of about 5 to 25 ml per gram of carvedilol and more preferably about 10 ml per gram of carvedilol.

The product obtained is filtered and dried to get pure carvedilol. The carvedilol obtained by the crystallization according to this procedure is almost free from bis impurity. The crystalline carvedilol so obtained has purity of more than 99.50% (By HPLC).

The following non-limiting examples presented to illustrate the best mode of carrying out the process of the present invention. The examples are not limited to the particular embodiments illustrated herein but include the permutations, which are obvious set forth in the description.

### Examples

### Example ― 1: Preparation of PTSA salt of carvedilol

a) In a dry reaction flask, 120 gm (0.502 moles) of 4-(2,3-epoxypropoxy)carbazole of Formula II, 188.7 gm (1.13 mole) of 2 - (2-methoxy phenoxy) ethylamine, and 1200 ml dimethylsulphoxide (DMSO) were charged under dry nitrogen atmosphere. The reaction mass was heated to about 70 °C till completion of reaction (about 20 hours), and then the reaction mass was cooled to 30 °C and 1200 ml water was added to it. The crude product was extracted with dichloromethane (1200 ml), and the dichloromethane layer was washed with water. The dichloromethane layer was mixed with 240 ml water, followed by the addition of 55.8 gm p-toluene sulphonic acid (PTSA) to attain a pH in the range of 7 to 8. After stirring, the layers were separated and the organic layer was washed with water.
b) Above organic layer was taken in a reaction flask and to it PTSA (about 130 gm) was added to get a pH of the reaction mass about 4-5. After stirring, the PTSA salt of carvedilol was filtered and washed with dichloromethane. The wet cake is dried to get 215 gm of carvedilol.PTSA salt.

### Example -2: Preparation of Crude carvedilol

190 gm of Carvedilol PTSA salt was taken in a flask, which was mixed with 1600 ml ethyl acetate, and 10% aqueous sodium carbonate solution (about 950 ml) till the pH is basic. The ethyl acetate layer was separated and distilled under vacuum to obtain a residue. 250 ml Toluene was added to the resulting residue after distillation of ethyl acetate and the precipitate obtained was filtered and dried to obtain 118 gm crude Carvedilol (88% yield).

### Example -3: Purification of Carvedilol

110 gm of crude carvedilol was dissolved in about 1300 ml of ethyl acetate at elevated temperature of 76 to 80°C to obtain a clear solution and optionally the clear solution was treated with charcoal and filtered to remove the insoluble. The ethyl acetate solution was concentrated to about 1/3^{rd} and cooled the solution to a temperature of 20-30°C to precipitate pure carvedilol. The obtained crystals were filtered off and dried to obtain 88 gm of pure carvedilol (80%).

### Example -4: Preparation of carvedilol

In a dry reaction flask charged 25.0 g 4 - (2,3-epoxy propoxy) carbazole (0.104 moles), 39.35 g of 2 - (2- methoxy phenoxy) ethylamine (0.235moles) in 250 ml dimethyl sulfoxide. The temperature of the reaction mass was raised to about 70°C under stirring and maintaining the reaction mixture at 68 - 72°C for 18 - 20 hrs. The reaction mass was cooled to about 30°C and quenched the reaction mass in 250 ml water, stirred and extracted the resultant solution in 250 ml dichloromethane. The organic layer was separated and washed with aqueous sulphuric acid till pH of the washings about 7.0 - 8.0. The organic layer was separated and further adjusted the pH with the aqueous sulfuric acid to 4.0 - 4.5 to precipitate carvedilol sulphate salt. The precipitated salt was filtered and taken in 300 ml ethyl acetate and made alkaline with 10% sodium carbonate solution. The reaction mass was stirred and separated the organic layer. The ethyl acetate was distilled under vacuum, and 330 ml toluene was added to it. The solid obtained was filtered and crystallized from ethyl acetate to obtain pure carvedilol. Yield = 58 - 62%.

## Claims

1. A process for the production of carvedilol (Formula 1) comprising: 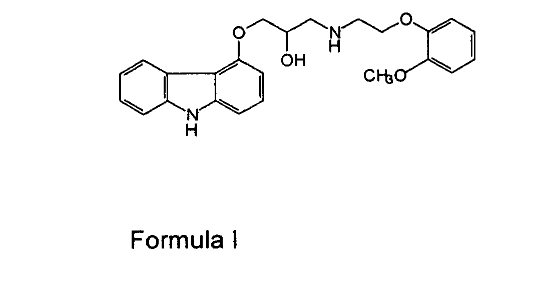
a. reacting the 4- (2,3- epoxypropoxy)carbazole (Formula-II) and 2-(2-methoxyphenoxy)ethylamine (Formula- III) in DMSO; and 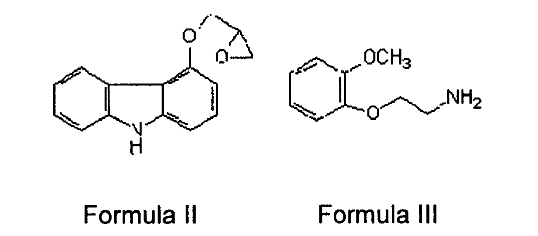
b. recovering carvedilol substantially free of the bis impurity 1,1-[{2-(2-methoxyphenoxy)ethyl}nitrilo]bis[3-(9*H*-carbazol-4-yloxy)propan-2-ol] impurity (Formula IV):

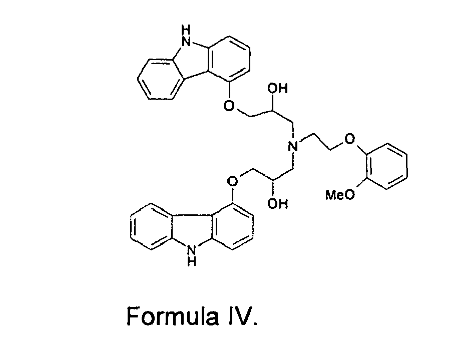

2. The process as claimed in claim 1 wherein the recovery of carvedilol further comprises:
a. forming a solution of crude carvedilol in an organic solvent;
b. washing said solution with an aqueous acid until the pH of the washings is in the range of from 7 to 8;
c. adjusting the pH of the carvedilol reaction solution to a pH of from 4 to <7 with an acid selectively to form a carvedilol acid salt substantially free of the bis impurity; and
d. transforming the carvedilol acid salt into carvedilol base substantially free of the bis-impurity.

3. The process as claimed in claim 2, wherein the organic solvent is a chlorinated hydrocarbon.

4. The process as claimed in claim 3, wherein the chlorinated hydrocarbon is selected from dichloromethane or ethylene chloride.

5. The process as claimed in any one of claims 2 to 4, wherein said aqueous acid is selected from hydrochloric acid, sulphuric acid, p-toluene sulphonic acid, acetic acid and phosphoric acid.

6. The process as claimed in any one of claims 2 to 5, wherein said carvedilol salt is selected from sulphate, p-toluene sulphonate, acetate, and phosphate.

7. The process as claimed in claim 6, wherein the carvedilol salt is p-toluene sulphonate salt.

8. The process as claimed in any one of claims 1 to 7, wherein the starting 2 - (2- methoxy phenoxy) ethylamine is used in molar excess relative to 4- (2,3- epoxy propoxy)carbazole.

9. The process as claimed in any one of claims 1 to 8, wherein the molar ratio of 2 - (2- methoxy phenoxy) ethylamine is in the range of 1.5 to 2.5 relative to the 4- (2,3- epoxy propoxy)carbazole employed.

10. The process as claimed in any one of claims 1 to 9, wherein the recovery of carvedilol comprises subjecting the crude carvedilol to repeated leaching by toluene or water or their mixture thereof.

11. The process as claimed in any one of claism 1 to 10, wherein the carvedilol is further purified by crystallization from ethyl acetate.

## Patentansprüche

1. Verfahren zur Herstellung von Carvedilol (Formel I), umfassend: 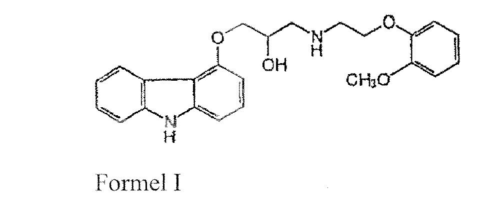
a. zur Reaktion bringen des 4-(2,3-Epoxypropoxy)carbazols (Formel II) und 2-(2-Methoxyphenoxy)ethylamins (Formel III) in DMSO; und 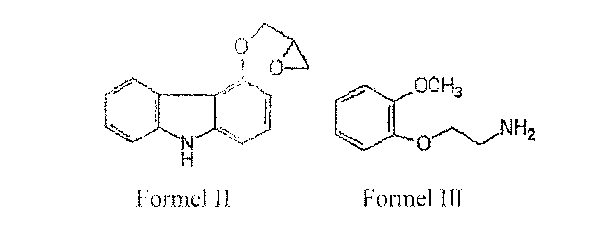
b. Rückgewinnen von Carvedilol, im Wesentlichen frei von der Bis-Unreinheit 1,1-[{2-(2-Methoxyphenoxy)ethyl}nitrilo]bis[3-(9*H*-carbazol-4-yl-oxy)propan-2-ol]-Unreinheit (Formel IV):

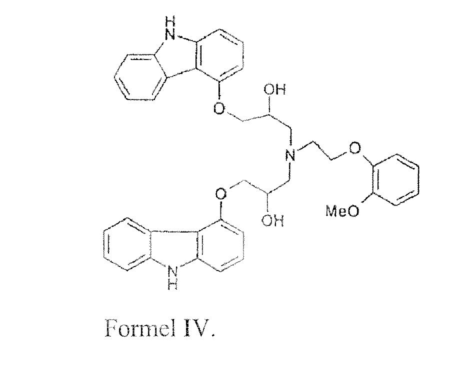

2. Verfahren nach Anspruch 1, worin die Rückgewinnung von Carvedilol ferner Folgendes umfasst:
a. Bilden einer Lösung aus rohem Carvedilol in einem organischen Lösungsmittel;
b. Waschen der Lösung mit einer wässrigen Säure, bis der pH der Waschflüssigkeit im Bereich von 7 bis 8 liegt;
c. Einstellen des pHs der Carvedilol-Reaktionslösung auf einen pH von 4 bis <7 mit einer Säure, um selektiv ein Carvedilol-Säuresalz zu bilden, das im Wesentlichen frei von der Bis-Unreinheit ist; und
d. Umwandeln des Carvedilol-Säuresalzes in eine Carvedilol-Base, die im Wesentlichen frei von der Bis-Unreinheit ist.

3. Verfahren nach Anspruch 2, worin das organische Lösungsmittel einen chlorierten Kohlenwasserstoff darstellt.

4. Verfahren nach Anspruch 3, worin der chlorierte Kohlenwasserstoff aus Dichlormethan oder Ethylenchlorid ausgewählt ist.

5. Verfahren nach einem der Ansprüche 2 bis 4, worin die wässrige Säure aus Salzsäure, Schwefelsäure, p-Toluensutfonsäure, Essigsäure und Phosphorsäure ausgewählt ist.

6. Verfahren nach einem der Ansprüche 2 bis 5, worin das Carvedilol-Salz aus Sulfat, p-Toluensulfonat, Acetat und Phosphat ausgewählt ist.

7. Verfahren nach Anspruch 6, worin das Carvedilol-Salz für p-Toluensulfonatsalz steht.

8. Verfahren nach einem der Ansprüche 1 bis 7, worin das Ausgangs-2-(2-Methoxyphenoxy)ethylamin in einem molaren Überschuss bezogen auf 4-(2,3-Epoxypropoxy)carbazol verwendet wird.

9. Verfahren nach einem der Ansprüche 1 bis 8, worin das Molverhältnis von 2-(2-Methoxyphenoxy)ethylamin im Bereich von 1,5 bis 2,5 bezogen auf das 4-(2,3-Epoxypropoxy)carbazol liegt.

10. Verfahren nach einem der Ansprüche 1 bis 9, worin die Rückgewinnung von Carvedilol das Aussetzen des rohen Carvedilols dem wiederholten Auslaugen mit Toluen oder Wasser oder einem Gemisch davon umfasst.

11. Verfahren nach einem der Ansprüche 1 bis 10, worin das Carvedilol ferner durch Kristallisation aus Ethylacetat gereinigt wird.

## Revendications

1. Procédé de production de carvedilol (Formule 1) comprenant : 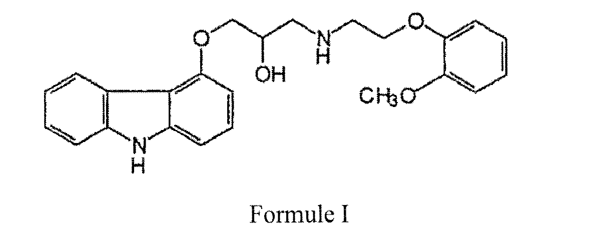
a. la réaction du 4-(2,3-époxypropoxy)carbazole (Formule II) et de la 2-(2-méthoxyphénoxy)éthylamine (Formule III) dans du DMSO ; et 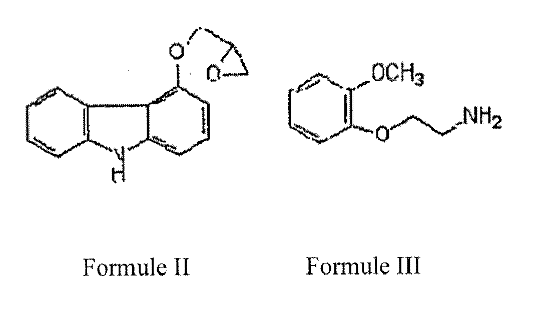
b. la récupération du carvedilol sensiblement dépourvu de l'impureté bis, l'impureté 1,1-[{2-(2-méthoxyphénoxy)éthyl}nitrilo]bis[3-(9H-carbazol-4-yloxy)-propan-2-ol] (Formule IV) :

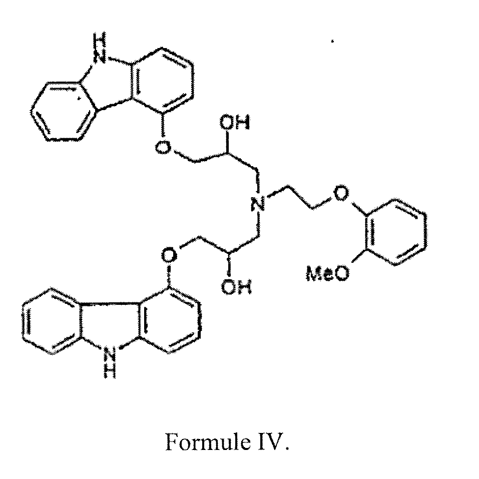

2. Procédé selon la revendication 1, dans lequel la récupération du carvedilol comprend en outre :
a. la formation d'une solution de carvedilol brut dans un solvant organique ;
b. le lavage de ladite solution par un acide aqueux jusqu'à ce que le pH des eaux de lavage se trouve dans la plage de 7 à 8 ;
c. l'ajustement du pH de la solution réactionnelle de carvedilol jusqu'à un pH de 4 à <7 par un acide pour former sélectivement un sel acide de carvedilol sensiblement dépourvu de l'impureté bis ; et
d. la transformation du sel acide de carvedilol en base de carvedilol sensiblement dépourvue de l'impureté bis.

3. Procédé selon la revendication 2, dans lequel le solvant organique est un hydrocarbure chloré.

4. Procédé selon la revendication 3, dans lequel l'hydrocarbure chloré est choisi parmi le dichlorométhane ou le chlorure d'éthylène.

5. Procédé selon l'une quelconque des revendications 2 à 4, dans lequel ledit acide aqueux est choisi parmi l'acide chlorhydrique, l'acide sulfurique, l'acide p-toluènesulfonique, l'acide acétique et l'acide phosphorique.

6. Procédé selon l'une quelconque des revendications 2 à 5, dans lequel ledit sel de carvedilol est choisi parmi le sulfate, le p-toluènesulfonate, l'acétate et le phosphate.

7. Procédé selon la revendication 6, dans lequel le sel de carvedilol est le sel de p-toluènesulfonate.

8. Procédé selon l'une quelconque des revendications 1 à 7, dans lequel la 2-(2-méthoxyphénoxy)éthylamine de départ est utilisée en excès molaire par rapport au 4-(2,3-époxypropoxy)carbazole.

9. Procédé selon l'une quelconque des revendications 1 à 8, dans lequel le rapport molaire de la 2-(2-méthoxyphénoxy)éthylamine se trouve dans la plage allant de 1,5 à 2,5 par rapport au 4-(2,3-époxypropoxy)carbazole employé.

10. Procédé selon l'une quelconque des revendications 1 à 9, dans lequel la récupération de carvedilol comprend la soumission du carvedilol brut à une lixiviation répétée par du toluène ou de l'eau ou un mélange de ceux-ci.

11. Procédé selon l'une quelconque des revendications 1 à 10, dans lequel le carvedilol est purifié davantage par cristallisation dans l'acétate d'éthyle.
